# EUROPEAN PATENT APPLICATION

(11) **EP 2 264 067 A1**
(43) Date of publication of application: **22.12.2010**
(21) Application number: 09305567.1
(22) Date of filing: 18.06.2009
(51) Int. Cl.: C07K 14/705, A61K 39/00

(54) **HLA-G alpha 1 multimers and pharmaceutical uses thereof**

(71) Applicant: Hla-G Technologies, 69005 Lyon (FR)
(72) Inventor: Rulleau, Laurence, Quebec, Laval H7E3E3 (CA); Martin, Jacques-François, 69160 Tassin la demi-lune (FR)
(74) Representative: Becker, Philippe

(57) **Abstract**

The present invention relates to alpha 1 multimers and the uses thereof. The invention also relates to methods of producing such multimers, pharmaceutical compositions comprising the same, as well as their uses for treating various diseases including organ/tissue rejection.

## Description

The present invention relates to novel multimers and pharmaceutical uses thereof. The invention more specifically relates to multimers of alpha 1 polypeptides of an HLA-G antigen. The invention also relates to methods of producing such multimers, pharmaceutical compositions comprising the same, as well as their uses for treating various diseases including organ/tissue rejection.

### BACKGROUND

Major histocompatibility complex (MHC) antigens are divided up into three main classes, namely class I antigens, class II antigens (HLA-DP, HLA-DQ and HLA-DR), and class III antigens.

Class I antigens comprise classical antigens, HLA-A, HLA-B and HLA-C, which exhibit 3 globular domains (α1, α2 and α3) associated with beta2 microglobulin, as well as non classical antigens HLA-E, HLA-F, and HLA-G.

HLA-G is a non-classic HLA Class I molecule expressed by extravillous trophoblasts of normal human placenta epithelial cells and cornea. HLA-G antigens are essentially expressed by the cytotrophoblastic cells of the placenta and function as immunomodulatory agents protecting the foetus from the maternal immune system (absence of rejection by the mother). The sequence of the HLA-G gene has been described (e.g., Geraghty et al. Proc. Natl. Acad. Sci. USA, 1987, 84, 9145-9149 ; Ellis; et al., J. Immunol., 1990, 144, 731-735) and comprises 4396 base pairs. This gene is composed of 8 exons, 7 introns and a 3' untranslated end, corresponding respectively to the following domains: exon 1: signal sequence, exon 2: alpha1 extracellular domain, exon 3: alpha2, extracellular domain, exon 4: alpha3 extracellular domain, exon 5: transmembrane region, exon 6: cytoplasmic domain I, exon 7: cytoplasmic domain II (untranslated), exon 8: cytoplasmic domain III (untranslated) and 3' untranslated region. Seven isoforms of HLA-G have been identified, among which 4 are membrane bound (HLA-G1, HLA-G2, HLA-G3 and HLA-G4) and 3 are soluble (HLA-G5, HLA-G6 and HLA-G7) (see e.g.,Carosella et al. Immunology Today 1996, vol. 17, p 407).

The mature HLA-G1 protein isoform comprises the three external domains (α1-α3), the transmembrane region and the cytoplasmic domain.

The HLA-G2 protein isoform does not comprise the α2 domain, i.e., the α1 and α3 domains are directly linked, followed by the transmembrane domain and the cytoplasmic domain.

The HLA-G3 protein isoform lacks both the α2 and α3 domains, i.e., it comprises the α1 domain directly linked to the transmembrane domain and the cytoplasmic domain. The HLA-G4 protein isoform lacks the α3 domain, i.e., it comprises the α1 domain, the α2 domain, the transmembrane domain and the cytoplasmic domain.

Soluble HLA-G isoforms all lack the transmembrane and cytoplasmic domains. More specifically:
The HLA-G5 protein isoform contains the α1, α2 and α3 domains, as well as an extra C-terminal peptide sequence of 21 amino acid residues encoded by intron 4 (as a result of intron 4 retention after transcript splicing and RNA maturation).
The HLA-G6 protein isoform corresponds to the HLA-G5 without α2, i.e., HLA-G6 contains α1 and α3 domains, as well as an extra C-terminal peptide sequence of 21 amino acid residues encoded by intron 4 (as a result of intron 4 retention after transcript splicing and RNA maturation
The HLA-G7 protein isoform contains only the alpha1 domain, as well as 2 additional C-terminal amino acid residues encoded by intron2 (as a result of intron 2 retention after transcript splicing and RNA maturation).

All of these isoforms have been described e.g., in Kirszenbaum M. et al., Proc. Natl. Acad. Sci. USA, 1994, 91, 4209-4213; European Application EP 0 677 582; Kirszenbaum M. et al., Human Immunol., 1995, 43, 237-241; Moreau P. et al., Human Immunol., 1995, 43, 231-236).

Previous studies have shown that HLA-G proteins are able to inhibit allogeneic responses such as proliferative T lymphocyte cell response, cytotoxic T lymphocytes mediated cytolysis, and NK cells mediated cytolysis (Rouas-Freiss N. et al., Proc. Natl. Acad. Sci., 1997, 94, 5249-5254 ; Proc. Natl. Acad. Sci., 1997, 94, 11520-11525; Semin Cancer Biol 1999, vol 9, p. 3). As a result, HLA-G-based procedures have been proposed for treating graft rejection in allogeneic or xenogenic organ/tissue transplantation. HLA-G proteins have also been proposed for the treatment of cancers (EP1 054 688), inflammatory disorders (EP1 189 627) and, more generally, immune related diseases. It has also been proposed to fuse HLA-G proteins to specific ligands in order to target HLA-G to particular cells or tissues (WO2007091078). It should be noted, however, that no results or experimental data have been provided to show that such targeting fusions are active.

HLA-G antigen appears to adopt a dimer conformation in vivo as a result of the formation of an intermolecular disulfide bridge between Cysteine residue 42 of the α1 domains of two HLA-G molecules (Apps et al., Eur. J. Immunol. 2007, vol. 37 p. 1924 ; W02007/011044). It has been proposed that receptor binding sites of HLA-G dimers are more accessible than those of corresponding monomers, so that dimers would have a higher affinity and slower dissociation rate than monomers. However, it is not clear what conformation is the most active for pharmaceutical purpose, which isoform is the most efficient, or how appropriate HLA-G dimers or oligomers may be produced.

### SUMMARY OF THE INVENTION

The present invention relates to multimers of HLA-G alpha 1 polypeptides, pharmaceutical compositions comprising the same, and the uses thereof. Unexpectedly, the invention shows that HLA-G alpha1 polypeptides, when properly assembled, can produce multimers having the ability to efficiently inhibit organ rejection in vivo. These multimers thus represent very valuable drug candidates for treating such disorders, as well as other immune-related diseases.

An object of this invention thus resides in a multimer comprising at least two alpha 1 polypeptides of an HLA-G antigen.

As will be discussed below, the alpha1 polypeptides may be linked together in different ways such as, without limitation, through disulfide bridging, a spacer group and/or a carrier.

A further object of this invention resides in a method of producing a multimer as defined above, the method comprising mixing alpha1 polypeptides under conditions allowing multimerisation and, optionally, separating multimers from free polypeptides (i.e., monomers).

The invention also relates to a polypeptide of SEQ ID NO: 1, as well as to an isolated nucleic acid encoding such a polypeptide and corresponding vector and recombinant cells.

A further object of this invention is a pharmaceutical composition comprising a multimer as defined above or obtainable by the above method.

A further object of this invention is a pharmaceutical composition comprising a polypeptide of SEQ ID NO: 1.

The invention further relates to multimers, polypeptide or pharmaceutical compositions as defined above for treating organ or tissue rejection, inflammatory diseases or auto-immune diseases.

A further objet of this invention also relates to a method of treating organ/tissue rejection, the method comprising administering to a subject in need thereof an effective amount of a multimer, polypeptide or composition of this invention. More specifically, the method comprises administering the multimer, polypeptide or composition to the subject, prior to, during and/or after tissue/organ transplant.

A further object of this invention is a method of promoting tolerance to graft in a subject, the method comprising administering to a subject in need thereof an effective amount of a multimer, polypeptide or composition as defined above.

The invention may be used in any mammalian subject, preferably in human subjects. As will be further disclosed below, the multimers of this invention are able to substantially inhibit tissue rejection in vivo following transplantation.

### LEGEND TO THE FIGURES

Figure 1: Graft survival in mice following administration of alpha1 multimers comprising antibody mediated alpha1 coated beads.
Figure 2: Graft survival in mice following administration of alpha1 multimers comprising directly coated alpha1 beads.
Figure 3: Graft survival in mice following administration of alpha1 multimers. Blue: control group with beads only. Orange: single injection of directly coated alpha1 beads.
Yellow: single injection of antibody mediated alpha1 coated beads. Green: two injections of directly coated alpha1 beads. Salmon: two injections of antibody mediated alpha1 coated beads.

### DETAILED DESCRIPTION OF THE INVENTION

The present invention relates to multimers comprising several HLA-G alpha 1 polypeptides and the uses thereof. The multimers of this invention have been shown to effectively inhibit graft rejection in vivo. More specifically, the inventors have surprisingly found that alpha1 polypeptides, when correctly assembled in multimers, have the ability to induce efficient immune tolerance in vivo.

As discussed above, HLA-G antigens function as immunomodulatory agents protecting the foetus from the maternal immune system. Various HLA-G isoforms have been reported, which are either membrane-bound or soluble. These isoforms contain distinct functional domains, selected from extracellular globular domains, designated α1, α2 and α3, a trans-membrane domain and a cytoplasmic domain. While the biological activity and mechanism of action of certain HLA-G isoforms (such as mature HLA-G1) have been documented, the relative contribution of each domain to the immunoregulatory activity, especially in soluble form, has not been studied in detail.

In this regard, it has been documented that the inhibitory activity of HLA-G antigen is mediated by binding to ILT inhibitory receptors ILT2 or ILT4. More specifically, it has been proposed that such binding occurs through the alpha3 domain of HLA-G (Shiroishi et al., PNAS 103 (2006) 16412). Guillard et al. (Molecular Immunology 45 (2008) 419) have also suggested a role of the alpha1 domain in the activation of NFKB. Such an effect, however, is mediated by binding to KIR-type receptors and is distinct from the inhibitory activity of HLA-G, which is mediated by the ILT2 or ILT4 receptor.

The inventors have now observed that alpha1 polypeptides, in multimers, are able to protect graft rejection in vivo. In this context, the receptor capable of interacting with HLA-G is the murine inhibitory receptor PIRB (homologous to human ILT-4). However, this receptor is known to interact with the alpha 3 domain of HLA-G. Similarly, in human in vitro experiments the effects are not due to interaction with ILT-2 or ILT-4 since these receptors interact with the alpha 3 domain of HLA-G. Without being bound by theory, the inventors believe the unexpected results obtained could be explained by the existence of unknown inhibitory receptors that bind alpha1 multimers and induce an immune tolerance (as observed in vivo), or by the fact that alpha1 multimers adopt a novel and unexpected quaternary structure which allows their interaction with ILT receptors.

The results obtained show that the multimers of this invention exhibit high immunoregulatory activity in vivo and therefore represent efficient drugs for treating immune-related disorders, particularly for reducing unwanted or deleterious immune responses in a subject. The results obtained more specifically show that multimers of this invention can induce a 100% or even more increase in graft survival in vivo compared to placebo.

A first object of this invention thus resides in a multimer comprising at least two alpha 1 polypeptides.

Within the context of the present invention, the term "alpha1 polypeptide" designates a polypeptide comprising the amino acid sequence of an alpha1 domain of an HLA-G antigen, or a functional fragment thereof, and essentially devoid of other functional HLA-G domains. More preferably, the alpha1 polypeptide comprises the amino acid sequence of an alpha1 domain of a HLA-G antigen. In a multimer of this invention, it is preferred that all alpha1 monomers have the same amino acid sequence. However, it is also contemplated that alpha1 polypeptides having different sequences are present in a multimer of this invention.

More preferably, the alpha 1 polypeptide comprises the amino acid sequence of the α1 domain of an HLA-G antigen, or a functional fragment thereof, and lacks functional α2, α3, TM and cytoplasmic domains of an HLA-G antigen.

The alpha1 domain of HLA-G is encoded by exon 2, and corresponds to amino acids 1-90 of mature human HLA-G. The amino acid sequence of the α1 domain can thus be derived directly from the publications of Geraghty et al. quoted above, or Ellis et al., J. Immunol., 1990, 144, 731-735. This sequence is also available on line (see for instance Genebank numbers for HLA-G: first cloning of genomic sequence: Geraghty et al, PNAS 1987: PubMed ID : 3480534, GeneID: 3135 ; First cloning of HLA-G1 cDNA : Ellis et al Journal of Immunology 1990. PubMed ID : 2295808). Furthermore, the sequences of HLA-G5, HLA-G6 and HLA-G7 are also available from US5,856,442, US6,291,659, FR2,810,047, or Paul et al., Hum. Immunol 2000; 61: 1138, from which the sequence of the alpha1 domain can be obtained directly.

Even more preferably, the alpha 1 polypeptide comprises the amino acid sequence of the α1 domain of an HLA-G antigen, or a functional fragment thereof, and contains less than 20, more preferably less than 15, even most preferably less than 10 or 5 additional amino acids which flank the alpha1 domain in a native HLA-G isoform.

A particular example of an alpha1 polypeptide of this invention is a polypeptide consisting of the sequence of an alpha1 domain of an HLA-G antigen, or a functional fragment thereof.

In a specific embodiment, the alpha 1 polypeptide consists essentially of amino acids 1-90 of a mature HLA-G antigen, or a functional fragment thereof.

The sequence of a preferred alpha1 polypeptide is provided in SEQ ID NO: 1, which represents a particular object of this invention.

A "functional fragment" designates a fragment which retains the ability to induce graft tolerance in vivo when used as a multimer of this invention. More preferably, a functional fragment comprises at least 20, more preferably at least 30, 40 or 50 consecutive amino acids of the alpha1 domain. In a typical embodiment, the functional fragment contains at least 60 consecutive amino acids of the alpha 1 domain. The functionality of the fragment may be verified as disclosed in the experimental section. In particular, the functionality may be verified by preparing a multimer of the fragments, administering the multimer to an animal model prior to organ/tissue transplantation, and verifying the graft survival rate. Where the multimer extends the duration of graft survival by 50%, as compared to placebo, the fragment may be considered as functional.

In a specific embodiment of the invention, the alpha 1 polypeptide is a polypeptide of SEQ ID NO: 1, or a functional fragment thereof comprising at least 50 consecutive amino acids of SEQ ID NO: 1.

It should be understood that natural variants of HLA-G antigens exist, e.g., as a result of polymorphism, which are included in the present application. Also, variants of the above sequences which contain certain (e.g., between 1 and 10, preferably from 1 to 5, most preferably 1, 2, 3, 4 or 5) amino acid substitutions or insertions are also included in the present invention.

Alpha1 polypeptides of this invention may be produced by techniques known per se in the art, such as recombinant techniques, enzymatic techniques or artificial synthesis. In a preferred embodiment, the alpha1 polypeptides are produced by artificial synthesis using known chemistry and synthesisers. The alpha1 polypeptides may comprise either natural amino acids, or non-natural or modified amino acid residues. They may be in L and/or D conformation. The polypeptides may comprise either amine linkages and/or modified, peptidomimetic linkages. Also, the polypeptides may be terminally protected and/or modified, e.g., through chemical or physical alteration of lateral functions, for instance.

As indicated above, the invention relates to multimers of alpha1 polypeptides.

Within the context of the present invention, the term "multimer" designates a molecule (or a composition or product) comprising at least two alpha1 polypeptides (monomers) as defined above, associated together. The term multimer thus includes dimers, as well as molecules comprising 3, 4, 5, 6, 7 or even more alpha1 monomers. Multimers of this invention may comprise up to 100, 500, 1000 or even more alpha1 monomers. Furthermore, the multimers of this invention may contain other monomers, in addition to said at least two alpha1 polypeptides. In particular, multimers of the present invention may contain at least two alpha1 monomers and a heteromonomer. In a specific embodiment, multimers of this invention contain alpha1 polypeptides only.

A particular example of a multimer of this invention is a dimer. In this respect, in a specific embodiment, the invention relates to an alpha1 dimer.

Within multimers of this invention, the various monomers may be linked together in different manner such as, without limitation, through disulfide bridging (especially for a dimer), or through a spacer group and/or a carrier. In a preferred embodiment, the alpha1 polypeptides are linked covalently or through an affinity interaction.

In a particular embodiment, the invention relates to an alpha1 dimer comprising two alpha1 polypeptides linked through a disulfide bridge. More specifically, the two alpha1 polypeptides are linked through a disulfide bridge between cystein residues at amino acid position 42 in human HLA-G antigens.

In a further particular embodiment, the alpha1 polypeptides (or monomers) are linked through a spacer or a carrier. In a particular embodiment, monomers are linked to a carrier, thereby producing a multimer. The carrier can be of different nature. It is preferably biocompatible, and most preferably biologically inert. The carrier may be a molecule, such as a protein, e.g., albumin (e.g., human serum albumin), or an inert solid carrier, such as a bead. The bead may be made of (or covered with) any biocompatible material, such a glass, metal, a polymer, coral, etc. In a particular embodiment, the carrier is a bead having a mean diameter below 50µm, more preferably below 10µm, typically of about 5 µm or less. The monomers may be linked to the carrier through different types of coupling reactions, such as affinity interaction or the use of functional groups. Affinity interaction may be obtained by coating the carrier with ligands that bind alpha1 polypeptides (e.g., antibodies or fragments thereof). Affinity interaction may also be obtained by adding to the alpha1 polypeptides and to the carrier, respectively a member of a binding pair (e.g., avidin and biotin). Coupling may also be obtained through bi-functional groups such as maleimide, etc. Furthermore, it should be noted that multimers may contain monomers linked to a carrier and further engaged in inter-molecular disulfide bridging.

In a particular embodiment, a multimer of this invention is a molecule comprising two or more alpha1 polypeptides linked to a carrier.

The multimers of this invention can be produced by various techniques. As discussed above, the monomers may be coupled together through different coupling techniques, such as covalent linkage (e.g., difulfide bridge, bi-functional group, etc) or affinity reaction.

For the production of a multimer through disulfide linkage, alpha1 polypeptides comprising a lateral SH group are contacted in solution, under conditions allowing formation of a disulfide linkage and, preferably, the dimers or multimers are separated. Multimers may be separated from monomers, e.g., on the basis of their molecular weight, e.g., by gel electrophoresis (such as PAGE). The suitable formation of multimers may also be verified using such method on aliquot samples, to measure the relative amount of multimer present in the solution and, if necessary, adjust the reaction condition. Conditions allowing formation of disulfide linkage include, for instance, a temperature of 10-30°C for 2-24 hours.

For the production of a multimer through the use of a carrier, the monomers are typically incubated in the presence of the carrier under conditions allowing attachment of the monomers on the carrier and, preferably, the multimer is separated. The carrier may be e.g., a solid carrier such as a bead, preferably a microbead. The carrier may also be a protein, such as serum-albumin. In order to facilitate interaction between the monomers and the carrier, the carrier may be functionalized to contain reactive groups able to interact with the monomers. As an example, the carrier may be coated with a ligand of alpha1 polypeptides, such as antibodies or fragments thereof (e.g., Fab fragments, CDR fragments, ScFv, etc) or a chemical coupling reagent (e.g.; maleimide). Alternatively, the carrier may be functionalized by a reactant able to bind a ligand of the alpha1 polypeptides. As an example, the carrier may be coated with an anti-human IgG Fc fragment, and the ligand may be a human polyclonal IgG directed against an HLA-G1 antigen. In such a case, the monomers, carrier and ligand may be incubated together, in order to allow proper association of the monomers to the beads.

In further embodiment, the carrier and monomers may be modified to contain cross-reactive groups (e.g., avidin and biotin). In such a case, incubation of the carrier and monomers will cause multimerisation on the carrier.

The multimer formed (i.e., the complex between the carrier and the alpha1 polypeptide) can be isolated using various techniques known per se in the art, including centrifugation, sedimentation, electromagnetic separation, etc.

Specific examples of multimers of the invention are:
- dimers of alpha1 polypeptides of SEQ ID NO: 1 linked through disulfide bridge ;
- multimers of alpha1 polypeptides of SEQ ID NO: 1 linked to a carrier such as a microbead; and
- multimers of alpha1 polypeptides of SEQ ID NO: 1 obtained by the method as disclosed above.

As mentioned in the examples, these multimers are able to promote graft tolerance in vivo.

Furthermore, the polypeptide of SEQ ID NO: 1, as well as a nucleic acid molecule encoding a polypeptide of SEQ ID NO: 1, also represent specific objects of this invention. The invention indeed shows that the polypeptide of SEQ ID NO: 1 has substantial in vivo activity for treating graft rejection and may be used to prepare very active multimers.

The coding nucleic acid may be e.g., RNA or DNA, single- or double-stranded. It may be produced by techniques known per se in the art, such as genetic engineering, chemical or enzymatic synthesis, etc. In a particular embodiment, the nucleic acid further comprises a sequence encoding a peptide for secretion, operably linked to the sequence encoding the polypeptide. As a result, expression of such a nucleic acid leads to the secretion of the polypeptide by the selected host cell. The peptide permitting secretion may by of various origin, such as from human or mammalian genes, e.g., B2M, interleukin, HLA-G, etc.

A further object of this invention also resides in a vector comprising a nucleic acid as defined above. The vector may be a cloning and/or expression vector, such as a plasmid, cosmid, phage, a viral vector, an artificial chromosome, etc. Specific examples of such vectors include pFUSE plasmids, pUC plasmids, pcDNA plasmids, pBR plasmids, retroviral vectors, adenoviral vectors, baculoviral vectors, lambda phage vectors, etc. The vector may comprise regulatory sequences, such as a promoter, a terminator, an origin of replication, etc. The vector may be used to produce the polypeptide of this invention in vitro, by recombinant techniques, or directly in vivo, in gene therapy approaches.

A further object of this invention is a recombinant host cell comprising a nucleic acid or a vector as defined above. The host cell may be prokaryotic or eukaryotic. Examples of prokaryotic hosts include any bacteria, such as E. coli. Examples of eukaryotic cells include yeasts, fungi, mammalian cells, plant cells or insect cells. Recombinant cells of this invention may be prepared by transformation techniques known per se in the art, such as transfection, lipofection, electroporation, protoplast transformation, etc. These cells may be maintained and cultured in any suitable culture media.

Recombinant cells of this invention can be used e.g., to produce the polypeptide of this invention in vitro or ex vivo, or as cell therapy products, to produce the polypeptide in vivo.

In this respect, an object of this invention also resides in a method of producing a polypeptide of SEQ ID NO: 1, the method comprising culturing a recombinant host cell of the invention under conditions allowing expression of the nucleic acid molecule, and recovering the polypeptide produced. The polypeptide may be recovered and/or purified using techniques known per se in the art, such as centrifugation, filtration, chromatographic techniques, etc.

A further object of this invention is a pharmaceutical composition comprising a multimer as defined above or obtainable by a method as disclosed above and, preferably, a pharmaceutically acceptable excipient or carrier.

A further object of this invention is a pharmaceutical composition comprising a polypeptide of SEQ ID NO: 1 and, preferably, a pharmaceutically acceptable excipient or carrier.

Suitable excipients or carriers include any pharmaceutically acceptable vehicle such as buffering agents, stabilizing agents, diluents, salts, preservatives, emulsifying agents, sweeteners, etc. The excipient typically comprises an isotonic aqueous or non aqueous solution, which may be prepared according to known techniques. Suitable solutions include buffered solutes, such as phosphate buffered solution, chloride solutions, Ringer's solution, and the like. The pharmaceutical preparation is typically in the form of an injectable composition, preferably a liquid injectable composition, although other forms may be contemplated as well, such as tablets, gelules, capsules, syrups, etc. The compositions of this invention may be administered by a number of different routes, such as by systemic, parenteral, oral, rectal, nasal or vaginal route. They are preferably administered by injection, such as intravenous, intraarterial, intramuscular, intraperitoneal, or subcutaneous injection. Transdermal administration is also contemplated. The specific dosage can be adjusted by the skilled artisan, depending on the pathological condition, the subject, the duration of treatment, the presence of other active ingredients, etc. Typically, the compositions comprise unit doses of between 10ng and 100 mg of multimer, more preferably between 1 µg and 50 mg, even more preferably between 100 µg and 50 mg. The compositions of the present invention are preferably administered in effective amounts, i.e., in amounts which are, over time, sufficient to at least reduce or prevent disease progression. In this regard, the compositions of this invention are preferably used in amounts which allow the reduction of a deleterious or unwanted immune response in a subject.

As mentioned above, the multimers of this invention have strong immune-regulatory activity and may be used to treat a variety of disease conditions associated with abnormal or unwanted immune response. More specifically, the multimers of this invention are suitable for treating immune-related disorders such as, particularly, organ or tissue rejection, inflammatory diseases or auto-immune diseases.

As disclosed in the experimental section, the multimers of this invention can substantially inhibit allogeneic graft rejection in vivo.

An object of the present invention thus resides in a multimer, polypeptide or composition as disclosed above for treating graft rejection.

A further object of this invention resides in a method of treating graft rejection in a subject, the method comprising administering to a subject in need thereof an effective amount of a composition as disclosed above.

The term treating designates for instance the promotion of the graft tolerance within the receiving subject. The treatment can be performed prior to, during and/or after the graft, and may be used as an alternative therapy to existing immunosuppressive agents or, as a combined therapy with actual immunosuppressive agents. The invention is applicable to allogenic, semi-allogenic or even xenogenic transplantation, and may be used for any type of transplanted organs or tissues including, without limitation, solid tissues, liquid tissues or cells, including heart, skin, kidney, liver, lung, liver-kidney, etc.

A further object of this invention is an improved method for transplanting an organ or tissue in a subject, the improvement comprising administering to the subject, prior to, during and/or after transplantation, an effective amount of a composition as disclosed above.

A further object of this invention is a method for promoting graft tolerance in a subject, the method comprising administering to the subject, prior to, during and/or after transplantation, an effective amount of a composition as disclosed above.

A further object of this invention is a method for reducing graft rejection in a subject, the method comprising administering to the subject, prior to, during and/or after transplantation, an effective amount of a composition as disclosed above.

In a preferred embodiment, the composition is administered at least twice to the subject. Indeed, the results shown in this application demonstrate that a repeated administration leads to a further increased benefit, e.g., to a further significantly increased graft tolerance in vivo.

A further object of the present invention resides in a multimer, polypeptide or composition as disclosed above for treating an auto-immune disease. The invention also resides in a method of treating an autoimmune disease in a subject, the method comprising administering to a subject in need thereof an effective amount of a composition as disclosed above. The autoimmune disease may be Rheumatoid arthritis, Crohn's disease or multiple sclerosis. In such disease conditions, the invention allows to reduce the deleterious immune response which is responsible for the pathology.

Another object of the present invention resides in a multimer, polypeptide or composition as disclosed above for treating an inflammatory disease.

A further object of this invention resides in a method of treating an inflammatory disease in a subject, the method comprising administering to a subject in need thereof an effective amount of a composition as disclosed above.

It should be understood that the amount of the composition actually administered shall be determined and adapted by a physician, in the light of the relevant circumstances including the condition or conditions to be treated, the exact composition administered, the age, weight, and response of the individual patient, the severity of the patient's symptoms, and the chosen route of administration. Therefore, the above dosage ranges are intended to provide general guidance and support for the teachings herein, but are not intended to limit the scope of the invention.

Further aspects and advantages of this invention will be disclosed in the following examples, which should be considered as illustrative and not limiting the scope of this application.

### EXAMPLES

### Example 1: Preparation of an alpha1 polypeptide

The alpha1 polypeptide of SEQ ID NO: 1 was synthesised using a peptide synthesiser.

### Example 2: Alpha1 dimers through disulfide linkage

Alpha1 polypeptides are incubated with sample buffer containing dithiothreitol ("reduced") or not ("non-reduced"), boiling, electrophoresed on polyacrylamide gels and transferred onto Hybond ECL nitrocellulose membranes. Following incubation with non-fat milk in PBS 1X, the membrane is incubated overnight with an anti-HLA-G polyclonal antibody and revealed using HorseRadish peroxidase-conjugated goat antimouse secondary antibody. Membranes are revealed with ECL detection system (Amersham Pharmacia Biosciences).

Under the above conditions, alpha1 polypeptides form dimers, which can be identified e.g., by electrophoresis.

### Example 3: Production of Alpha1 multimers using a carrier

Sulfate latex beads (4%w/v 5µm, Invitrogen) were used as carrier. They were coated with alpha1 monomers either directly or indirectly, i.e., using anti-HLA-G antibody 4H84 (0.5mg/ml, BD Pharmingen).

For indirect coating, 10⁸ Sulfate latex beads were incubated with 20µg/ml purified anti-human HLA-G Antibody for 2hrs at 37°C, followed by 2hr incubation with BSA (2 mg/ml). After washing, the beads were incubated with 1µg/ml of HLA-G alpha1 peptide (90 mer, produced as in example 1) at 4°C for 16hrs.

To generate HLA-G peptide directly coated beads, 10⁸Sulfate latex beads were coated with 1µg/ml of HLA- G alpha1 peptide at 4°C for 16hrs, followed by 2hr incubation with BSA (2 mg/ml).

All beads were subsequently washed 2 times by 1xPBS. 5ml of HLA- G alpha1 peptide (1µg/ml) was used for 5x 10⁶ sulfate latex beads.

Such multimers of the invention were used to induce or increase graft tolerance in vivo (see example 4).

### Example 4: Effect of Alpha1 multimers on allogeneic skin transplantation

To evaluate the biological activity of the alpha-1 multimers of this invention, several studies were conducted *in vivo.*

Specific pathogen-free C57BL/6 (H-2b) mice (8-10 weeks of age) were used as skin graft recipients throughout the study. Recipient mice received alphal-coupled beads. Donor skin was from MHC class II-disparate B6.CH-2bm12 (bm12, H-2b) mice. Allogeneic skin grafts have been performed by standard methods. Briefly, skin (1.0 cm²) from the tail of donor mice (12-14 weeks old) was grafted onto the flank of recipient, anesthetized mice. The graft was covered with gauze and plaster, which was removed on day 10. Grafts were scored daily until rejection (defined as 80% of grafted tissue becoming necrotic and reduced in size). All skin grafting survival data were tested by Kaplan Meier Survival Analysis.

In a first series of experiments, alpha1 peptide-coated sulfate latex beads (5×10⁶) prepared as disclosed in example 3, were injected intraperitoneally on the day before skin grafting. As a negative control, sulfate latex beads were prepared in an identical manner except that 1xPBS or HeLa Negative Control was used rather than alpha 1 polypeptide.

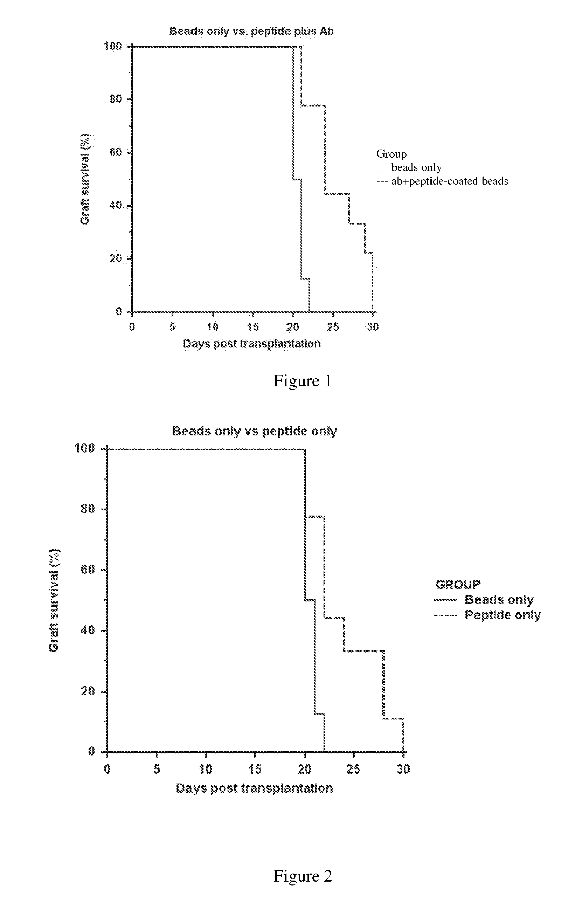

The results of these experiments are depicted on Figures 1 and 2. They show that alpha1 multimers of this invention were able to substantially improve graft tolerance in vivo. In particular, they show that the multimers are able to substantially improve mean survival, which is very surprising. More specifically, while mean survival is 22 days in non treated mice, it is 25 days in treated mice. Also, mice treated with the multimers prepared by indirect antibody-mediated coating showed an increased mean time graft survival of four days, as compared to directly coated beads.

It should be noted that each day of graft survival in the model corresponds to approximately at least one month of graft survival in human subjects, so that the compositions of this invention are believed to improve graft survival by at least several months in human subjects.

These results therefore show that mice (C57BL/6 (H2B)) treated once with the alpha1 multimer prior to allograft (B6.CH-2bm12 (bm12, H-2b)) exhibit an increased graft survival.

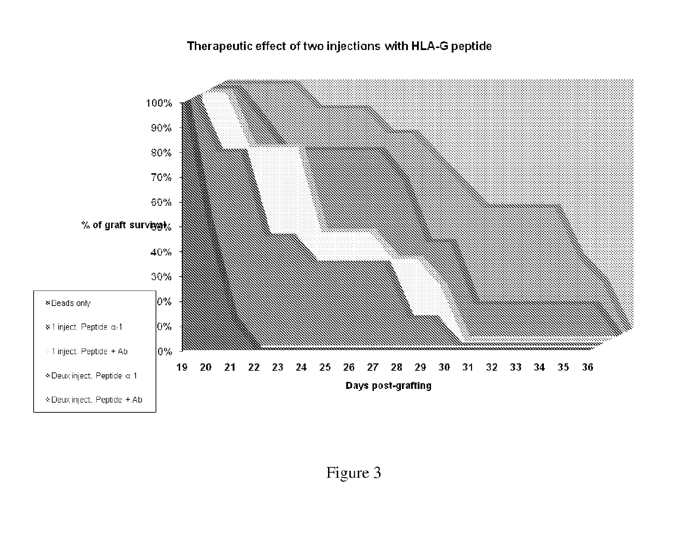

Additional studies conducted *in vivo* in wild type mice showed that two injections (24 hours prior to the graft and then 10 days post-grafting) of multimers of this invention increased the graft survival by six days as compared to a single administration. In Figure 3, the results of these additional experiments are presented, and demonstrate a very strong and astonishing graft tolerance effect, leading to a more than 100% increase in graft survival.

These results therefore clearly illustrate and support the claimed use of alpha-1 multimers as a therapeutic product to improve graft survival.

SEQUENCE LISTING

G S H S M R Y F S A A V S R P G R G E
Gly Ser His Ser Met Arg Tyr Phe Ser Ala Ala Val Ser Arg Pro Gly Arg Gly Glu

P R F I A M G Y V D D T Q F V R F D S
Pro Arg Phe Ile Ala Met Gly Tyr Val Asp Asp Thr Gln Phe Val Arg Phe Asp Ser

D S A C P R M E P R A P W V E Q E G P
Asp Ser Ala Cys Pro Arg Met Glu Pro Arg Ala Pro Trp Val Glu Gln Glu Gly Pro

E Y W E E E T R N T K A H A Q T D R M
Glu Tyr Trp Glu Glu Glu Thr Arg Asn Thr Lys Ala His Ala Gln Thr Asp Arg Met

N L Q T L R G Y Y N Q S E A
Asn Leu Gln Thr Leu Arg Gly Tyr Tyr Asn Gln Ser Glu Ala

SEQ ID NO :1

## Claims

1. A multimer comprising at least two alpha 1 polypeptides of an HLA-G antigen.

2. The multimer of claim 1, wherein said at least two alpha 1 polypeptides are linked through a disulfide bride.

3. The multimer of claim 1, wherein said at least two alpha 1 polypeptides are linked through a carrier or a spacer group.

4. The multimer of any one of the preceding claims, which comprises at least three alpha 1 polypeptides, preferably at least 4, 5, 6 or 7 alpha 1 polypeptides.

5. The multimer of any one of the preceding claims, wherein said alpha 1 polypeptide comprises the amino acid sequence of the α1 domain of an HLA-G antigen, or a functional fragment thereof, and lacks functional α2, α3, TM and cytoplasmic domains of an HLA-G antigen.

6. The multimer of anyone of the preceding claims, wherein said alpha 1 polypeptide consists essentially of amino acids 1-90 of a mature HLA-G antigen, or a functional fragment thereof comprising at least 50 amino acids.

7. The multimer of anyone of the preceding claims, wherein said alpha 1 polypeptide is a polypeptide of SEQ ID NO: 1, or a functional fragment thereof comprising at least 50 consecutive amino acids of SEQ ID NO: 1.

8. The multimer of claim 3, wherein said carrier is a polypeptide, such as human serum albumin, or a bead.

9. A dimer of an alpha1 polypeptide.

10. A method of producing a multimer of any one of claims 1 to 9, the method comprising mixing alpha 1 polypeptides under conditions allowing their multimerisation, and collecting multimers.

11. The method of claim 10, wherein said polypeptides are mixed in the presence of a carrier, preferably micro-beads or human serum-albumin, and the multimers are separated.

12. The method of claim 11, wherein the carrier is a micro-bead coated with an affinity reagent which binds alpha1 polypeptides.

13. A pharmaceutical composition comprising a multimer of any one of claims 1 to 9 or obtainable by a method of anyone of claims 10-12.

14. The pharmaceutical composition of claim 13, for treating organ or tissue rejection.

15. The pharmaceutical composition of claim 13, for treating an inflammatory disease or an auto-immune disease.

16. A polypeptide of SEQ ID NO: 1.
